# EUROPÄISCHE PATENTSCHRIFT

(11) **EP 0 923 381 B1**
(45) Veröffentlichungstag und Bekanntmachung des Hinweises auf die Patenterteilung: **14.04.2004**
(21) Anmeldenummer: 96930139.9
(22) Anmeldetag: 30.08.1996
(51) Int. Cl.: A61K 35/22, C07J 75/00

(54) **VERFAHREN ZUR GEWINNUNG VON OESTROGENEN AUS STUTENHARN**
METHOD TO OBTAIN OESTROGENS FROM MARE'S URINE
PROCEDE D'OBTENTION D'OESTROGENES A PARTIR D'URINE DE JUMENT

(43) Veröffentlichungstag der Anmeldung: 23.06.1999
(73) Patentinhaber: Solvay Pharmaceuticals GmbH, 30173 Hannover (DE)
(72) Erfinder: BAN, Ivan, D-30539 Hannover (DE); HEINEMANN, Henning, D-31275 Lehrte (DE); MECHTOLD, Gerhard, D-30627 Hannover (DE); RASCHE, Heinz-Helmer, D-31303 Burgdorf (DE)
(74) Vertreter: Gosmann, Martin
(86) Internationale Anmeldenummer: PCT/EP1996/003820
(87) Internationale Veröffentlichungsnummer: WO 1998/008526

(56) Entgegenhaltungen:
- DE-A- 2 150 819
- CHEMICAL ABSTRACTS, vol. 104, no. 11, 17.März 1986 Columbus, Ohio, US; abstract no. 82747, SEMENKA JIRI ET AL: "Sorbent for isolation of 17-oxosteroids from biological materials" Seite 141; Spalte 1; XP002030907 & CS-A-224881, 1-08-1985
- STEROIDS, Bd. 11, Nr. 3, 1968, Seiten 265-272, XP002030905 BRADLOW H.L. : "Extraction of steroid conjugates with a neutral resin"
- STEROIDS, Bd. 8, Nr. 2, 1966, Seiten 195-204, XP002030906 GUPTA D. ET AL: "A sephadex extraction of urinary steroid conjugates"

## Beschreibung

Die vorliegende Erfindung betrifft die Gewinnung eines natürlichen Gemisches konjugierter Oestrogene aus dem Harn trächtiger Stuten.

Oestrogene werden in der Medizin zur Hormonsubstitutionstherapie eingesetzt. Insbesondere werden Oestrogengemische zur Behandlung und Prophylaxe der bei Frauen auftretenden Beschwerden der Wechseljahre nach natürlicher oder artifizieller Menopause eingesetzt. Hierbei haben sich natürliche Gemische konjugierter Oestrogene, wie sie im Harn trächtiger Stuten vorliegen, als besonders wirksam und gut verträglich erwiesen.

Der gelöste Feststoffgehalt im Harn trächtiger Stuten (= pregnant mares urine, im folgenden abgekürzt als "PMU") kann natürlicherweise in weiten Bereichen schwanken und im allgemeinen in einem Bereich von 40 - 90 g Trockensubstanz pro Liter liegen. Neben Harnstoff und sonstigen üblichen Harninhaltsstoffen sind in dem Feststoffgehalt des PMU phenolische Bestandteile in Mengen von ca. 2 - 5 Gew.-% bezogen auf Trockensubstanz enthalten. Unter diesen phenolischen Bestandteilen befinden sich Kresole und das als HPMF bekannte Dihydro-3,4-bis[(3-hydroxyphenyl)methyl]-2(3H)-furanon. Diese können in freier oder konjugierter Form vorliegen. In dem PMU ist ein natürliches Gemisch von Oestrogenen enthalten, welches weitgehend in konjugierter Form, z. B. als Schwefelsäurehalbester-Natriumsalz (im folgenden abgekürzt als "Sulfatsalz"), vorliegt. Der Gehalt an konjugierten Oestrogenen (berechnet als Oestrogensulfatsalz) kann zwischen 0,3 und 1 Gew.-% bezogen auf Trockensubstanz betragen.

Üblicherweise werden konjugierte Oestrogene enthaltende Extrakte aus dem PMU durch Extraktion mit einem polaren mit Wasser nicht oder nur wenig mischbaren organischen Lösemittel wie beispielsweise Essigsäureethylester, n-Butanol oder Cyclohexanol gewonnen. Bei derartigen Flüssig-Flüssig-Extraktionen treten jedoch eine Vielzahl von Problemen, wie starke Schaumbildung, Sedimentbildung, Emulsionsbildung und schlechte Phasentrennung auf. Es werden im allgemeinen mehrere Extraktionsschritte benötigt, was zu Verlusten und einer nur teilweisen Gewinnung des Oestrogengehaltes führt.

Von H. L. Bradlow wurde 1968 (siehe Steroids 11 (1968), 265-272) vorgeschlagen zur Extraktion von konjugierten Oestrogenen aus Harn Amberlite XAD-2^{R}, ein neutrales unpolares hydrophobes Polystyrolharz der Fa. Rohm und Haas, einzusetzen. Die angegebene Adsorptionskapazität ist gering. Nach Bradlow wird ein gegebenenfalls verdünnter Harn mit einer niedrigen Durchflußgeschwindigkeit durch eine das Harz enthaltende Säule geleitet. Die Oestrogene werden mit Methanol oder Ethanol eluiert. Es werden jedoch keine Angaben über die übrigen in dem oestrogenhaltigen Eluat enthaltenen Stoffe gemacht.

Aufgabe der vorliegenden Erfindung ist es, ein technisches Verfahren zur Gewinnung des natürlichen Gemisches konjugierter Oestrogene aus dem PMU unter Vermeidung der von den bisher üblichen Flüssig-Flüssig-Extraktionen bekannten Nachteile zu entwikkeln, welches ein an phenolischen Harninhaltsstoffen abgereichertes weitgehend Kresol- und HPMF-freies Produkt liefert.

Es wurde nun ein Verfahren gefunden, mit welchem in einer Festphasen-Extraktion an einem nichtionischen semipolaren polymeren Adsorberharz ein an phenolischen Harninhaltsstoffen abgereichertes, weitgehend Kresol- und HPMF-freies, jedoch den natürlichen Oestrogen-Gehalt des PMU praktisch vollständig enthaltendes Gemisch gewonnen werden kann, welches als Ausgangsmaterial für die Herstellung von das natürliche Gemisch konjugierter Oestrogene aus dem PMU als Wirkkomponente enthaltenden Pharmazeutika dienen kann.

Das erfindungsgemäße Verfahren zur Gewinnung eines an phenolischen Harninhaltsststoffen abgereicherten natürlichen Gemisches konjugierter Oestrogene aus dem PMU ist dadurch gekennzeichnet, daß man
a) eine Harnflüssigkeit, welche den von Schleim- und Feststoffen befreiten Harn, ein eingeengtes Konzentrat dieses Harns oder ein durch Membranfiltration dieses Harns erhaltenes eingeengtes Harnretentat darstellt, mit einer zur Adsorption des in der Harnflüssigkeit enthaltenen Gemisches konjugierter Oestrogene ausreichenden Menge eines semipolaren polymeren Adsorberharzes kontaktiert und ein mit dem Gemisch konjugierter Oestrogene beladenes semipolares polymeres Adsorberharz von der übrigen Harnflüssigkeit abtrennt,
b) das mit dem Gemisch konjugierter Oestrogene beladene semipolare polymere Adsorberharz mit einem auf einen pH-Bereich von mindestens 12,0 insbesondere von 12,5 - 14, eingestellten Waschwasser wäscht,
c) das gewaschene Adsorberharz mit einer zur Desorption des daran adsorbierten Gemisches konjugierter Oestrogene ausreichenden Menge einer Elutionsflüssigkeit, welche ein mit Wasser mischbares organisches Lösemittel aus der Gruppe mit Wasser mischbarer Ether, niederer Alkanole und niederer aliphatischer Ketone oder ein Gemisch aus dem mit Wasser mischbaren organischen Lösemittel und gegebenenfalls alkalisch eingestelltem Wasser darstellt, kontaktiert und ein das natürliche Gemisch konjugierter Oestrogene enthaltendes Eluat von dem Adsorberharz abtrennt und gegebenenfalls einengt.

Für das erfindungsgemäße Verfahren können der PMU als solcher, ein daraus durch Einengen gewonnenes Konzentrat oder ein daraus durch Membranfiltration gewonnenes Retentat eingesetzt werden. Der gesammelte Harn wird zunächst auf an sich bekannte Weise von Schleim- und Feststoffen befreit. Zweckmäßig läßt man Fest- und Schleimstoffe absetzen und trennt diese dann nach an sich bekannten Trennungsmethoden, beispielsweise Dekantieren, Separieren und/oder Filtrieren ab. So kann der PMU beispielsweise über eine an sich bekannte Trenneinrichtung, z. B. einen Separator, eine Filtrationsanlage oder einen Sedimentator geleitet werden. Als Trenneinrichtung kann z. B. ein Sandbett dienen, oder es können handelsübliche Separatoren, z. B. Düsen- oder Kammerseparatoren, eingesetzt werden. Gewünschtenfalls können auch eine Microfiltrationsanlage oder eine Ultrafiltrationsanlage eingesetzt werden, bei deren Verwendung gleichzeitig eine weitgehende Keim- und Virenfreiheit des filtrierten PMU erreicht werden kann.

Gewünschtenfalls können dem Harn Konservierungsmittel, Germizide, Bakterizide und/oder Anthelmintika zugesetzt werden.

Sofern anstelle des PMU ein aufkonzentriertes PMU-Retentat eingesetzt werden soll, kann dieses aus dem PMU durch an sich bekannte Membranfiltration gewonnen werden. Der Feststoffgehalt des Retentates und dessen Zusammensetzung können je nach dem eingesetzten PMU und der zur Membranfiltration verwendeten Membran, beispielsweise deren Porenweite, sowie den Bedingungen der Filtration variieren. Beispielsweise kann bei Verwendung einer Nanofiltrationsmembran eine nahezu verlustfreie Konzentration des Oestrogengehaltes in dem PMU-Retentat bei gleichzeitiger Entfernung von bis zu 50 Gew.-% der niedermolekularen PMU-Inhaltsstoffe erreicht werden. Für das erfindungsgemäße Verfahren können PMU-Retentate eingesetzt werden, welche bis zu einem Verhältnis von ca. 1:10, beispielsweise einem Verhältnis von ca. 1:7 aufkonzentriert wurden und deren Volumen somit bis auf ca. 1/10, beispielsweise ca. 1/7, des ursprünglichen PMU-Volumens eingeengt sein kann.

Die in dem Verfahrensschritt a) einsetzbaren semipolaren polymeren Adsorberharze sind poröse organische nichtionische Polymere, welche im Gegensatz zu unpolaren hydrophoben polymeren Adsorberharzen eine intermediäre Polarität (= z. B. mit einem Dipolmoment der aktiven Oberfläche des Harzes im Bereich von 1,0 bis 3,0, insbesondere 1,5 bis 2,0, Debye) und eine etwas hydrophylere Struktur besitzen, beispielsweise Polycarbonsäureesterharze. Zweckmäßig werden makroporöse semipolare Harze mit vorzugsweise makroretikularer Struktur und mit durchschnittlichen Porendurchmessern im Bereich von 50 bis 150, vorzugsweise 70 bis 100, Angström und einer spezifischen Oberfläche im Bereich von 300 bis 900, vorzugsweise 400 bis 500, m²/g eingesetzt. Als besonders geeignet erweisen sich makroporöse quervernetzte aliphatische Polycarbonsäureesterharze, insbesondere quervernetzte Polyacrylesterharze wie z. B. Amberlite XAD-7^{R} der Fa. Rohm und Haas.

Erfindungsgemäß kann die Adsorption der konjugierten Oestrogene an das semipolare Adsorberharz durch Kontaktierung des PMU oder dessen Retentats mit dem Adsorberharz erfolgen, indem die Harnflüssigkeit in einen das Adsorberharz enthaltenden Reaktor eingeführt und darin für eine zur Adsorption des Oestrogengehaltes ausreichende Zeit mit dem Adsorberharz in Kontakt gehalten wird. Nach erfolgter Adsorption der konjugierten Oestrogene an das semipolare Adsorberharz kann das mit dem Gemisch konjugierter Oestrogene beladene Adsorberharz von der übrigen Harnflüssigkeit auf an sich bekannte Weise abgetrennt werden. Zweckmäßigerweise kann die Harnflüssikgeit durch eine das Adsorberharz enthaltende Säule mit solcher Durchlaufgeschwindigkeit geleitet werden, daß die Kontaktzeit zur Adsorption des Oestrogengehaltes ausreicht. Geeignet sind beispielsweise Durchlaufgeschwindigkeiten, welche einem Durchlauf von 3 bis 10, vorzugsweise 5 bis 7, Vol.-Teilen PMU/1 Vol.-Teil Adsorberharz/-Stunde entsprechen. Die Adsorption wird bevorzugt bei Raumtemperatur durchgeführt. Zweckmäßig kann die Durchlaufgeschwindigkeit der Harnflüssigkeit durch den Reaktor durch Arbeiten bei geringem Überdruck oder Unterdruck gesteuert werden. Die Menge an anzuwendendem semipolarem Adsorberharz kann je nach Art des verwendeten Adsorberharzes und Menge des Feststoff-Gehaltes in der Harnflüssigkeit variieren. Bei Verwendung von PMU kann beispielsweise ein Volumenteil Adsorberharz, z. B. quervernetztes aliphatisches Polycarbonsäureester-Adsorberharz, mit bis zu 80 Volumen-Teilen vorbehandeltem PMU beladen werden, ohne daß merkliche Mengen Oestrogen in der abfließenden Harnflüssigkgeit nachweisbar sind. Bei Verwendung eines PMU-Konzentrates oder PMU-Retentates reduziert sich die Beladungskapazität des Adsorberharzes natürlich in dem Maße, in dem diese aufkonzentriert sind. So kann beispielsweise 1 Volumenteil an quervernetztem aliphatischem Polycarbonsäureester-Adsorberharz mit einer 20 bis 80, vorzugsweise 30 bis 50, Volumenteilen PMU entsprechenden Menge Harnflüssigkeit beladen werden.

Das mit dem Gemisch konjugierter Oestrogene beladene semipolare Adsorberharz wird in Verfahrensschritt b) mit einem auf einen pH-Bereich von mindestens 12,0, insbesondere von 12,5 bis 14, vorzugsweise ca. 12,5 bis 13,5, eingestellten Waschwasser gewaschen. Als Waschwasser können wäßrige Lösungen von in der Harnflüssigkeit löslichen inerten basischen Substanzen, welche stark genug sind, um einen pH-Wert von mindestens 12,5 zu erreichen, eingesetzt werden. Als gegenüber dem semipolaren polymeren Adsorberharz inerte, wasserlösliche basische Substanzen eignen sich vorzugsweise wasserlösliche anorganische Basen wie Alkalimetall- oder Erdalkalimetallhydroxide, insbesondere Natriumhydroxid. Zweckmäßig enthält das Waschwasser nur etwa diejenige Menge an basischen Substanzen, welche zum Erreichen des gewünschten pH-Wertes, vorzugsweise ca. pH 13, benötigt wird. Die Menge an Waschwasser wird so gewählt, daß sie ausreicht, um phenolische Harninhaltsstoffe weitgehend zu entfernen, ohne daß dabei nennenswerte Mengen konjugierter Oestrogene mit ausgewaschen werden. Als zweckmäßig erweist sich beispielsweise die Verwendung von 2 bis 10, insbesondere 4 bis 6, Bettvolumen Waschflüssigkeit pro Bettvolumen Adsorberharz. Hierbei wird das Waschwasser zweckmäßigerweise durch einen das Adsorberharz enhaltenden Reaktor mit einer Durchflußgeschwindigkeit von 3 bis 10, vorzugsweise 5 bis 7, Vol.-Teilen Waschwasser /1 Vol.-Teil Adsorberharz/Stunde geleitet.

Im Verfahrensschritt c) wird anschließend das gewaschene mit dem Gemisch konjugierter Oestrogene beladene Adsorberharz mit einer zur Elution des Gemisches der konjugierten Oestrogene ausreichenden Menge einer Elutionsflüssigkeit behandelt und ein das natürliche Gemisch der konjugierten Oestrogene des PMU enthaltendes Eluat gewonnen. Die erfindungsgemäß verwendete Elutionsflüssigkeit stellt ein mit Wasser mischbares organisches Lösemittel aus der Gruppe mit Wasser mischbarer Ether, niederer Alkanole und niederer aliphatischer Ketone oder ein Gemisch aus einem solchen mit Wasser mischbaren Lösemittel und gegebenenfalls alkalisch gestelltem Wasser dar. Als Etherbestandteile der Elutionsflüssigkeit eignen sich mit Wasser mischbare cyclische Ether wie Tetrahydrofuran oder Dioxan, aber auch mit Wasser mischbare offenkettige Ether wie beispielsweise Ethylenglycoldimethylether (= Monoglyme), Diethylenglycoldimethylether (= Diglyme) oder Ethyloxyethyloxyethanol (= Carbitol). Als niedere Alkanole eignen sich mit Wasser mischbare Alkylalkohole mit 1 - 4, vorzugsweise 1 - 3, Kohlenstoffatomen, insbesondere Ethanol oder Isopropanol. Als niedere aliphatische Ketone eignen sich mit Wasser mischbare Ketone mit 3 - 5 Kohlenstoffatomen, insbesondere Aceton. Als besonders günstig erweisen sich Elutionsflüssigkeiten, in denen das organische Lösemittel Ethanol ist. Zweckmäßig werden als Elutionsflüssigkeiten Gemische aus einem der vorgenannten mit Wasser mischbaren organischen Lösemittel und gegebenenfalls alkalisch eingestelltem Wasser eingesetzt. Der pH-Wert derartiger wasserhaltiger Elutionsmittel liegt im neutralen bis alkalischen Bereich bis zu pH 13 und kann vorteilhaft ca. 10 bis 12 betragen. Als Lösemittelkomponente in der wasserhaltigen Elutionsflüssigkeit wird ein in dem eingesetzten pH-Bereich stabiles Lösemittel ausgewählt. In wasserhaltigen alkalischen Elutionsflüssigkeiten mit pH-Werten von ca. 10 bis 12 eignen sich als Lösemittelkomponente niedere Alkanole, vorzugsweise Ethanol. Der gewünschte pH-Wert der wasserhaltigen Elutionsmittel wird durch Zugabe einer entsprechenden Menge einer wasserlöslichen inerten basischen Substanz, vorzugsweise einer anorganischen Base, beispielsweise eines Alkalimetalloder Erdalkalimetallhydroxides, insbesondere Natriumhydroxid, eingestellt. In wasserhaltigen Elutionsflüssigkeiten kann ein Volumenverhältnis von mit Wasser mischbarem organischem Lösemittel zu Wasser im Bereich von 40:60 bis 20:80, vorzugsweise ca. 30:70 vorliegen._Die eingesetzte Menge an Elutionsmittel kann ca. 3 bis 10, insbesondere ca. 4 bis 6, Bettvolumen pro Bettvolumen Adsorberharz betragen. Zweckmäßigerweise wird die Elutionsflüssigkeit durch einen das mit dem Oestrogengemisch beladene Adsorberharz enthaltenden Reaktor mit einer solchen Durchlaufgeschwindigkeit geleitet, daß die Kontaktzeit zur vollständigen Elution des Gemisches konjugierter Oestrogene ausreicht. Bei Verwendung eines Gemisches von Ethanol mit Wasser im Vol.-Verhältnis 30:70 eignen sich beispielsweise Durchlaufgeschwindigkeiten von 3 bis 10, vorzugsweise 5 bis 7, Vol.-Teilen Elutionsflüssigkeit pro 1 Vol.-Teil Adsorberharz pro Stunde. Zweckmäßig wird die Elution in einem Temperaturbereich von Raumtemperatur bis ca. 60 °C, vorzugsweise bei ca. 40 bis 50 °C, durchgeführt. Gewünschtenfalls wird die Durchlaufgeschwindigkeit durch Arbeiten bei leicht erhöhtem Druck, z. B. bei einem Überdruck von bis zu 0,2 bar, reguliert und das Eluat in mehreren Fraktionen aufgefangen. Die Gehalte der einzelnen Eluatfraktionen an konjugierten Oestrogenen und phenolischen Harninhaltsstoffen wie Kresolen und HPMF können auf an sich bekannte Weise durch Hochleistungsflüssigkeits-Chromatographie (= high performance liquid chromatography, abgekürzt als "HPLC") bestimmt werden.

Bei der Elution wird zunächst eine schwachgefärbte bis farblose, praktisch oestrogenfreie Vorlauffraktion erhalten, deren Menge im allgemeinen ca. einem Bettvolumen entspricht. Die Hauptmenge der konjugierten Oestrogene, beispielsweise zwischen 80 und 99 % der in dem Ausgangs-PMU vorliegenden konjugierten Oestrogene, befindet sich in den nachfolgenden dunkelgelb-braun gefärbten Haupteluatfraktionen, deren Menge im allgemeinen 2 bis 4 Bettvolumen umfaßt. In den nachfolgenden Nachlauffraktionen sind im allgemeinen nur noch Spuren an konjugierten Oestrogenen enthalten. Sofern Nachfolgefraktionen erhalten werden, welche noch einem Gehalt an konjugierten Oestrogenen von über 10 Gew.-% bezogen auf Trockensubstanz und weniger als 0,6 Gew.-% bezogen auf Trockensubstanz an Kresolen und HPMF enthalten, können diese mit dem oestrogenreichen Haupteluat zur Weiterverarbeitung vereinigt werden.

Das auf die vorstehend beschriebene Weise von dem Adsorberharz abgetrennte Haupteluat enthält das im PMU auftretende natürliche Gemisch konjugierter Oestrogene neben nur einem geringen Anteil des ursprünglich in dem PMU vorhandenen Gehaltes an phenolischen Harninhaltsstoffen. Dieses Eluat kann als Ausgangsmaterial für die Herstellung von das natürliche Gemisch konjugierter Oestrogene enthaltenden Arzneimitteln dienen. Gewünschtenfalls kann das Eluat auf an sich bekannte Weise weiter eingeengt werden, um ein weitgehend von organischem Lösemittel befreites, zur galenischen Weiterverarbeitung geeignetes Konzentrat zu erhalten. Gewünschtenfalls kann auch durch Sprühtrocknung ein elutionsmittelfreies Feststoffgemisch hergestellt werden. Sofern das natürliche Gemisch konjugierter Oestrogene für die Herstellung fester Arzneimittel verwendet werden soll, kann es zweckmäßig sein, dem die konjugierten Oestrogene enthaltenden Eluat einen festen Trägerstoff bereits vor einer Konzentrierung oder Sprühtrocknung zuzumischen, um auf diese Weise ein die konjugierten Oestrogene und Trägerstoffe enthaltendes Feststoffgemisch zu erhalten. Sowohl das das Oestrogengemisch enthaltende Eluat als auch ein daraus hergestelltes Konzentrat oder sprühgetrocknetes Feststoffprodukt können in an sich bekannter Weise in feste oder flüssige galenische Zubereitungen wie beispielsweise Tabletten, Dragees, Kapseln oder Emulsionen eingearbeitet werden. Diese galenischen Zubereitungen können nach an sich bekannten Methoden hergestellt werden unter Verwendung üblicher fester oder flüssiger Trägerstoffe wie z. B. Stärke, Cellulose, Milchzucker oder Talkum oder flüssigen Paraffinen und/oder unter Verwendung von üblichen pharmazeutischen Hilfsstoffen, beispielsweise Tablettensprengmitteln, Lösungsvermittlern oder Konservierungsmitteln. So kann das die konjugierten Oestrogene enthaltende Produkt mit den pharmazeutischen Träger- und Hilfsstoffen auf an sich bekannte Weise vermischt werden und das Gemisch in eine geeignete Dosierungsform überführt werden.

Die nachfolgenden Beispiele sollen die Erfindung näher erläutern, ohne jedoch ihren Umfang zu beschränken.

### Beispiele 1 - 3

Allgemeine Arbeitsvorschrift zur Gewinnung eines an phenolischen Harninhaltsstoffen weitgehend abgereicherten das natürliche Gemisch der im PMU enthaltenen konjugierten Oestrogene enthaltenden Extraktes aus PMU.

### A) Adsorption des Oestrogengehaltes des PMU an ein semipolares Polyacrylester-Adsorberharz.

Es wird eine Säule von 30 cm Höhe mit einem Durchmesser von 2,4 cm mit 65 ml eines in Wasser gequollenen semipolaren Polyacrylester-Adsorberharz (= Amberlite XAD-7 der Fa. Rohm und Haas, Korngröße 0,3 bis 1,2 mm, Dipolmoment 1,8 Debye, durchschnittlicher Porendurchmesser 80 Angström, spezifische Oberfläche ca. 450 m²/g trocken) gefüllt. 2 l eines durch eine Mikrofiltrationsanlage filtrierten oder durch Leiten durch einen Separator gereinigten PMU (Trockensubstanzgehalt (= TS) und auch mittels HPLC bestimmte Gehalte an Oestronsulfatsalz, Kresol und HPMF siehe nachfolgende Beispielstabelle) werden bei Raumtemperatur mit einer Durchflußgeschwindigkeit von 6 ml/min. (= ca. 5,5 Bettvolumen pro Stunde) durch die Säule geleitet. Der Oestrogengehalt des PMU ist vollständig auf der so beladenen semipolaren Adsorberharz-Säule adsorbiert. Die ablaufende Harnflüssigkeit wird mittels HPLC auf ihren Gehalt an konjugierten Oestrogenen (berechnet als Oestronsulfatsalz) untersucht und erweist sich als praktisch Oestrogenfrei. Der Ablauf wird verworfen.

### B) Waschen der beladenen Adsorberharz-Säule.

Die beladene Adsorberharz-Säule wird mit 300 ml einer wäßrigen Natriumhydroxidlösung mit dem in der Beispielstabelle angegebenen pH-Wert gewaschen. Hierzu wird das alkalische Waschwasser mit einer Durchflußgeschwindigkeit von 6 ml/min. (= ca. 5,5 Bettvolumen pro Stunde) durch die Säule geleitet. Die ablaufende Waschflüssigkeit wird mittels HPLC auf ihren Gehalt an Oestronsulfatsalz, Kresol und HPMF untersucht. Die Untersuchung zeigt, daß während der Waschphase weniger als 5 % der gesamten auf die Säule aufgegebenen Oestrogene ausgewaschen wird.

### C) Desorption der konjugierten Oestrogene von der gewaschenen Adsorberharz-Säule.

315 ml der Elutionsflüssigkeit (durch Zusatz von Natriumhydroxid alkalisch gestelltes Wasser/Lösemittel-Gemisch, Zusammensetzung und pH siehe nachfolgende Beispielstabelle) werden mit einer Fließgeschwindigkeit von ca. 6 ml/min. durch die auf die in der Beispielstabelle angegebene Elutionstemperatur vorgeheizte Säule geleitet. Das ablaufende Eluat wird in 6 Fraktionen aufgefangen. Die erste Fraktion beträgt ca. 65 ml (= ca. 1 Bettvolumen), die restlichen Fraktionen betragen jeweils ca. 50 ml. Die einzelnen Fraktionen werden mittels HPLC auf ihren Gehalt an Oestronsulfatsalz), Kresol und HPMF untersucht. Die erste Fraktion wird gesammelt, solange das Eluat farblos bis schwach gelb gefärbt erscheint. Diese Fraktion enthält nur Spuren an Oestrogensulfatsalz.

Nachdem das erste Bettvolumen an Eluat abgelaufen ist, tritt ein Farbwechsel des Eluates zu einem intensiven dunkelbraunen Farbton ein. In den nachfolgenden Fraktionen 2 bis 4 sind dann ca. 80 bis 98 % der gesamten auf der Säule adsorbierten Menge konjugierter Oestrogene enthalten. Die restlichen Fraktionen enthalten nur noch geringe Menge an Oestrogensulfatsalz. Dieses ist auch deutlich an der Abnahme der Farbintensität sichtbar. Gegebenenfalls können die Restfraktionen nach Abdestillieren des Lösemittelgehaltes nochmals dem Verfahrensschritt A) zugeführt werden.

Für die die Hauptmenge der konjugierten Oestrogene enthaltenden Fraktionen sind in der nachfolgenden Beispielstabelle jeweils der TS-Gehalt in Gew.-% und die durch HPLC bestimmten Gehalte an Oestronsulfatsalz, Kresol und HPMF angegeben. Diese Fraktionen stellen für eine galenische Weiterverarbeitung geeignete Extrakte dar.

### D) Regenerierung der Adsorberharz-Säule.

Zur Regenerierung wird die Säule zunächst mit 100 ml eines auf pH 12 eingestellten 50 % Ethanol enthaltenden Ethanol/-Wasser-Gemisches, dann mit 150 ml 10-%iger wäßriger Natriumcitratlösung und nochmals mit 150 ml des Ethanol/Wasser-Gemisches und schließlich mit 100 ml destilliertem Wasser gewaschen. Die gesamte Regeneration erfolgt bei einer Temperatur von 45 °C. Die Säule kann mehrmals, beispielsweise bis zu 40-mal, beladen und wieder regeneriert werden.

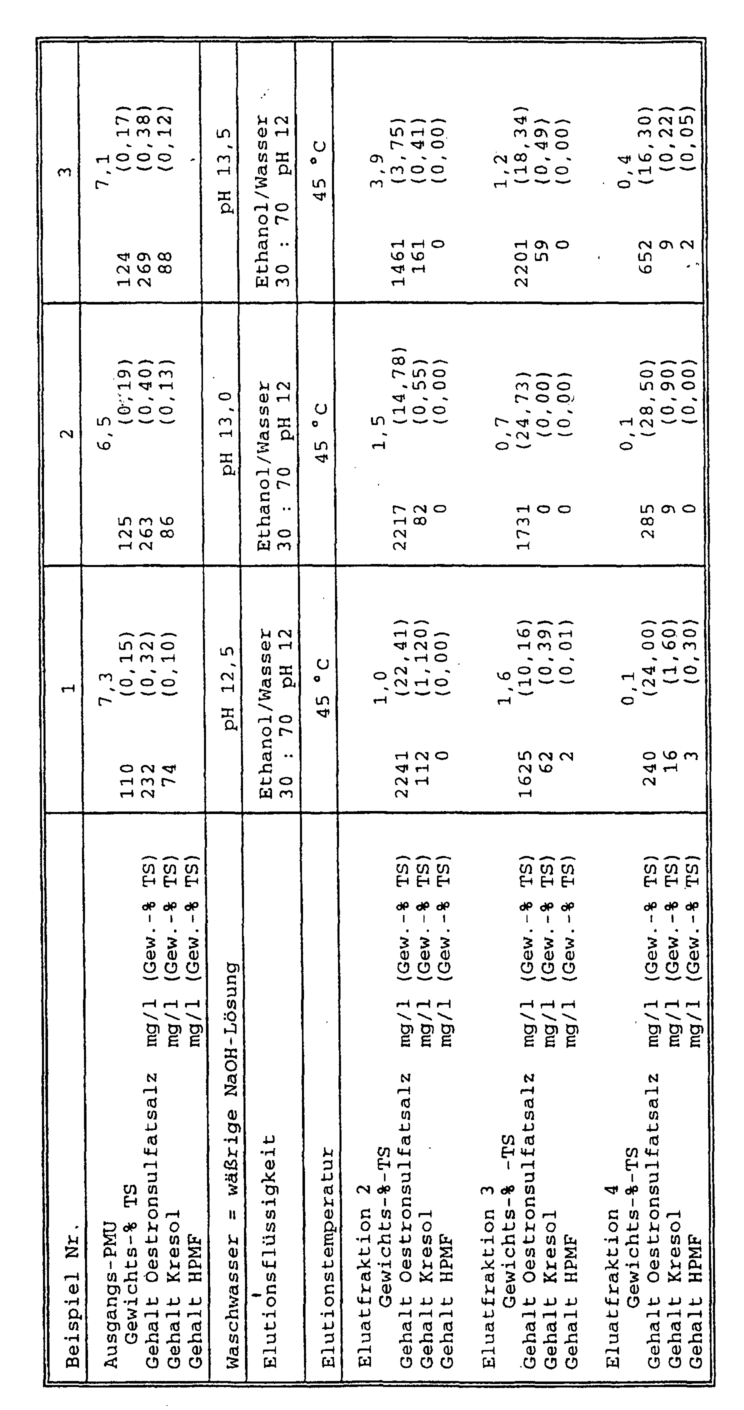

## Patentansprüche

1. Verfahren zur Gewinnung eines an phenolischen Harninhaltsstoffen abgereicherten natürlichen Gemisches konjugierter Oestrogene aus dem Harn trächtiger Stuten, **dadurch gekennzeichnet, daß** man
a) eine Harnflüssigkeit, welche den von Schleim- und Feststoffen befreiten Harn, ein eingeengtes Konzentrat dieses Harns oder ein durch Membranfiltration dieses Harns erhaltenes eingeengtes Harnretentat darstellt, mit einer zur Adsorption des in der Harnflüssigkeit enthaltenen Gemisches konjugierter Oestrogene ausreichenden Menge eines nichtionischen semipolaren polymeren Adsorberharzes kontaktiert und ein mit dem Gemisch konjugierter Oestrogene beladenes semipolares polymeres Adsorberharz von der übrigen Harnflüssigkeit abtrennt,
b) das mit dem Gemisch konjugierter Oestrogene beladene nichtionische semipolare polymere Adsorberharz mit einem auf einen pH-Bereich von mindestens 12,0 eingestellten Waschwasser wäscht, und
c) das gewaschene Adsorberharz mit einer zur Desorption des daran adsorbierten Gemisches konjugierter Oestrogene ausreichenden Menge einer Elutionsflüssigkeit, welche ein mit Wasser mischbares organisches Lösemittel aus der Gruppe mit Wasser mischbarer Ether, niederer Alkanole und niederer aliphatischer Ketone oder ein Gemisch aus dem mit Wasser mischbaren organischen Lösemittel und gegebenenfalls alkalisch eingestellter Wasser darstellt, kontaktiert und ein das natürliche Gemisch konjugierter Oestrogene enthaltendes Eluat von dem Aäsorberharz abtrennt und gegebenenfalls einengt.

2. Verfahren nach Anspruch 1, **dadurch gekennzeichnet, daß** im Verfahrensschritt a) als nichtionisches semipolares Adsorberharz ein makroporöses Polycarbonsäureesterharz eingesetzt wird.

3. Verfahren nach Anspruch 2, **dadurch gekennzeichnet, daß** als Polycarbonsäureesterharz ein quervernetztes aliphatisches Polycarbonsäureesterharz, vorzugsweise ein quervernetztes Polyacrylesterharz mit vorzugsweise makroretikulärer Struktur, eingesetzt wird.

4. Verfahren nach einem der vorstehenden Ansprüche, **dadurch gekennzeichnet, daß** in dem Verfahrensschritt a) 1 Volumenteil an nichtionischem semipolaren Adsorberharz mit einer 20 bis 80, vorzugsweise 30 bis 50, Volumenteilen Harn entsprechenden Menge Harnflüssigkeit beladen wird.

5. Verfahren nach einem der verstehenden Ansprüche, **dadurch gekennzeichnet, daß** in dem Verfahrensschritt a) die Harnflüssigkeit mit einer Durchflußgeschwindigkeit, welche einem Durchlauf von 3 bis 10, vorzugsweise 5 bis 7 Vol.-Teilen Harn/1 Vol.-Teil Adsorberharz/Stunde entspricht, durch einen das nichtionische semipolare polymere Adsorberharz enthaltenden Reaktor geleitet wird.

6. Verfahren nach einem der vorstehenden Ansprüche, **dadurch gekennzeichnet, daß** das im Verfahrensschritt b) eingesetzte Waschwasser eine auf ca. pH 12,5 bis 13,5 eingestellte wäßrige Natriumhydroxidlösung ist.

7. Verfahren nach einem der vorstehenden Ansprüche, **dadurch gekennzeichnet, daß** im Verfahrensschritt c) als Elutionsflüssigkeit ein Gemisch aus Wasser und einem mit Wasser mischbaren organischen Lösemittel mit einem Volumenverhältnis von organischem Lösemittel zu Wasser im Bereich von 20:80 bis 40:60, insbesondere 30:70 eingesetzt wird.

8. Verfahren nach einem der vorstehenden Ansprüche, **dadurch gekennzeichnet, daß** im Verfahrensschritt c) eine ethanolhaltige Elutionsflüssigkeit eingesetzt wird.

## Claims

1. A method for obtaining a natural mixture, depleted in phenolic urine contents, of conjugated oestrogens from the urine of pregnant mares, **characterised in that**
a) a urine, which represents the urine freed of mucilaginous substances and solids, a reduced concentrate of this urine or a reduced urine retentate obtained by membrane filtration of this urine, is contacted with a quantity of a non-ionic semi-polar polymeric adsorber resin sufficient for adsorption of the mixture of conjugated oestrogens contained in the urine, and a semi-polar polymeric adsorber resin laden with the mixture of conjugated oestrogens is separated off from the rest of the urine,
b) the non-ionic semi-polar polymeric adsorber resin laden with the mixture of conjugated oestrogens is washed with a washing water set to a pH range of at least 12.0, and
c) the washed adsorber resin is contacted with a quantity of an elution liquid sufficient for desorption of the mixture of conjugated oestrogens adsorbed thereon, which liquid represents a water-miscible organic solvent from the group of water-miscible ethers, lower alkanols and lower aliphatic ketones or a mixture of the water-miscible organic solvent and water which has optionally been rendered alkaline, and an eluate containing the natural mixture of conjugated oestrogens is separated off from the adsorber resin and optionally reduced.

2. A method according to Claim 1, **characterised in that** in method step a) a macroporous polycarboxylic acid ester resin is used as the non-ionic semi-polar adsorber resin.

3. A method according to Claim 2, **characterised in that** a cross-linked aliphatic polycarboxylic acid ester resin, preferably a cross-linked polyacrylic ester resin having a preferably macroreticular structure, is used as the polycarboxylic acid ester resin.

4. A method according to one of the preceding claims, **characterised in that** in method step a) 1 part by volume of non-ionic semi-polar adsorber resin is laden with a quantity of urine corresponding to 20 to 80, preferably 30 to 50, parts by volume urine.

5. A method according to one of the preceding claims, **characterised in that** in method step a) the urine is passed through a reactor containing the non-ionic semi-polar polymeric adsorber resin at a flow rate which corresponds to a throughput of 3 to 10, preferably 5 to 7, parts by volume urine/1 part by volume adsorber resin/hour.

6. A method according to one of the preceding claims, **characterised in that** the washing water used in method step b) is an aqueous sodium hydroxide solution set to approximately pH 12.5 to 13.5.

7. A method according to one of the preceding claims, **characterised in that** in method step c) a mixture of water and a water-miscible organic solvent having a volume ratio of organic solvent to water in the range of 20:80 to 40:60, in particular 30:70, is used as elution liquid.

8. A method according to one of the preceding claims, **characterised in that** in method step c) an ethanol-containing elution liquid is used.

## Revendications

1. Procédé d'obtention d'un mélange naturel d'oestrogènes conjugués, appauvri en substances urinaires phénoliques, à partir d'urine de juments gravides, **caractérisé en ce que**
a) on met en contact un liquide urinaire, qui représente l'urine exempte des mucosités et substances solides, un concentré de cette urine ou un rétentat urinaire concentré obtenu par filtration membranaire de cette urine, avec une quantité d'une résine d'adsorbeur polymère semi-polaire non ionique suffisante pour l'adsorption du mélange d'oestrogènes conjugués contenu dans le liquide urinaire, et on sépare du liquide urinaire restant une résine d'adsorbeur polymère semi-polaire chargée du mélange d'oestrogènes conjugués,
b) on lave la résine d'adsorbeur polymère semi-polaire non ionique chargée du mélange d'oestrogènes conjugués avec une eau de lavage ajustée à une gamme de pH d'au moins 12,0, et
c) on met en contact la résine d'adsorbeur lavée avec une quantité d'un liquide d'élution suffisante pour la désorption du mélange d'oestrogènes conjugués qui y est adsorbé, lequel liquide d'élution représente un solvant organique miscible à l'eau issu du groupe des éthers, des alcanols inférieurs et des cétones aliphatiques inférieures, miscibles à l'eau, ou un mélange du solvant organique miscible à l'eau et d'eau à pH éventuellement rendu alcalin, et on sépare un éluat contenant le mélange naturel d'oestrogènes conjugués de la résine d'adsorbeur, et on concentre éventuellement.

2. Procédé selon la revendication 1, **caractérisé en ce que**, dans l'étape de procédé a), on utilise comme résine d'adsorbeur semi-polaire non ionique une résine ester d'acide polycarboxylique macroporeuse.

3. Procédé selon la revendication 2, **caractérisé en ce que** l'on utilise en tant que résine ester d'acide polycarboxylique une résine ester d'acide polycarboxylique aliphatique réticulé, de préférence une résine de polyacrylester réticulé possédant de préférence une structure macroréticulaire.

4. Procédé selon l'une des revendications précédentes, **caractérisé en ce que**, dans l'étape de procédé a), on charge 1 partie en volume de résine d'adsorbeur semi-polaire non ionique avec une quantité de liquide urinaire correspondant à 20 à 80, de préférence 30 à 50, parties en volume d'urine.

5. Procédé selon l'une des revendications précédentes, **caractérisé en ce que**, dans l'étape de procédé a), le liquide urinaire est dirigé par un réacteur contenant la résine d'adsorbeur polymère semi-polaire non ionique selon une vitesse de passage qui correspond à un débit de 3 à 10, de préférence de 5 à 7, parties en volume d'urine/l partie en volume de résine d'adsorbeur/heure.

6. Procédé selon l'une des revendications précédentes, **caractérisé en ce que** l'eau de lavage utilisée dans l'étape de procédé b) est une solution aqueuse d'hydroxyde de sodium ajustée à un pH d'environ 12,5 à 13,5.

7. Procédé selon l'une des revendications précédentes, **caractérisé en ce que** dans l'étape de procédé c), on utilise en tant que liquide d'élution un mélange composé d'eau et d'un solvant organique miscible à l'eau selon un rapport de volume du solvant organique à l'eau dans la gamme allant de 20:80 à 40:60, en particulier égal à 30:70.

8. Procédé selon l'une des revendications précédentes, **caractérisé en ce que**, dans l'étape de procédé c), on utilise un liquide d'élution contenant de l'éthanol.
